(19) Europäisches Patentamt
European Patent Office
Office européen des brevets

(11) **EP 1 423 704 B1**

(12) **EUROPEAN PATENT SPECIFICATION**

(45) Date of publication and mention
of the grant of the patent:
**14.04.2010 Bulletin 2010/15**

(51) Int Cl.:
***G01N 33/68*** (2006.01)

(21) Application number: **02797965.7**

(22) Date of filing: **07.09.2002**

(86) International application number:
**PCT/EP2002/010057**

(87) International publication number:
**WO 2003/023405 (20.03.2003 Gazette 2003/12)**

(54) **DIAGNOSTIC AND THERAPEUTIC USE OF F-BOX PROTEINS FOR ALZHEIMER'S DISEASE AND RELATED NEURODEGENERATIVE DISORDERS**

DIAGNOSTISCHE UND THERAPEUTISCHE VERWENDUNG VON F-BOX-PROTEINEN BEI MORBUS ALZHEIMER UND VERWANDTEN NEURODEGENERATIVEN ERKRANKUNGEN

UTILISATION A DES FINS DIAGNOSTIQUES ET THERAPEUTIQUES DE PROTEINES A F-BOX POUR LA MALADIE D'ALZHEIMER ET LES TROUBLES NEURODEGENERATIFS ASSOCIES

(84) Designated Contracting States:
**AT BE BG CH CY CZ DE DK EE ES FI FR GB GR IE IT LI LU MC NL PT SE SK TR**

(30) Priority: **07.09.2001 EP 01121442**

(43) Date of publication of application:
**02.06.2004 Bulletin 2004/23**

(73) Proprietor: **Takeda Pharmaceutical Company Limited**
**Osaka 540-8645 (JP)**

(72) Inventors:
• **VON DER KAMMER, Heinz**
**22607 Hamburg (DE)**
• **POHLNER, Johannes**
**22175 Hamburg (DE)**
• **HIPFEL, Rainer**
**69126 Heidelberg (DE)**

(74) Representative: **von Kreisler Selting Werner**
**Deichmannhaus am Dom**
**Bahnhofsvorplatz 1**
**50667 Köln (DE)**

(56) References cited:
**WO-A-00/12679     WO-A-00/75328**

• **WINSTON J T ET AL: "A FAMILY OF MAMMALIAN F-BOX PROTEINS" CURRENT BIOLOGY, CURRENT SCIENCE,, GB, vol. 9, 1999, pages 1180-1182, XP000960309 ISSN: 0960-9822 cited in the application**
• **HOFMANN K ET AL: "A ubiquitin-interacting motif conserved in components of the proteasomal and lysosomal protein degradation systems" TIBS TRENDS IN BIOCHEMICAL SCIENCES, ELSEVIER PUBLICATION, CAMBRIDGE, EN, vol. 26, no. 6, 1 June 2001 (2001-06-01), pages 347-350, XP004245047 ISSN: 0968-0004**

**Description**

The present invention relates to methods of diagnosing, prognosticating and monitoring the progression of Alzheimer's disease in a subject. Furthermore, methods of therapy control and screening for modulating agents of Alzheimer's disease are provided.

Neurodegenerative diseases, in particular Alzheimer's disease (AD), have a severely debilitating impact on a patient's life. Furthermore, these diseases constitute an enormous health, social, and economic burden. AD is the most common age-related neurodegenerative condition affecting about 10 % of the population over 65 years of age and up to 45 % over age 85 (for a recent review see Vickers et al., Progress in Neurobiology 2000, 60:139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals suffering from AD are senile plaques, composed of amyloid-β protein, and profound cytoskeletal changes coinciding with the appearance of abnormal filamentous structures and the formation of neurofibrillary tangles. AD is a progressive disease that is associated with early deficits in memory formation and ultimately leads to the complete erosion of higher cognitive function. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10:184-192).

Currently, there is no cure for AD, nor is there an effective treatment to halt the progression of AD or even a method to diagnose AD ante-mortem with high probability. Several risk factors have been identified that predispose an individual to develop AD, among them most prominently the epsilon4 allele of apolipoprotein E (ApoE). Although there are rare examples of early-onset AD which have been attributed to genetic defects in the genes for APP, presenilin-1, and presenilin-2, the prevalent form of late-onset sporadic AD is of hitherto unknown etiologic origin. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurodegenerative diseases and to provide methods, materials, and animal models which are suited inter alia for the diagnosis, identification of compounds useful for therapeutic intervention, and development and monitoring of a treatment of these diseases. This object has been solved by the features of the independent claims of the present invention. The subclaims define preferred embodiments.

F-box proteins constitute a family of proteins characterized by an approximately 40 amino-acid motif called the F-box. The F-box motif was first recognized in the cyclin F protein (Bai et al., Cell 1996, 86:263-274). In subsequent studies, at least 38 human F-box proteins have been identified (Kipreos and Pagano, Genome Biology 2000, 1: 3002.1-3002.7; Cenciarelli, et al., Current Biology 1999, 9:1177-1179; Winston et al., Current Biology 1999, 9:1180-1182). Frequently, F-box proteins may contain further secondary motifs such as leucine zippers, zinc and ring fingers, cyclin domains, and proline-reach regions. Functionally, the F-box is a peptide module acting as a site of protein-protein interactions. In specific examples, the F-box domain allows proteins to enter into a complex with Skp1 (S-phase kinase-associated protein 1). F-box proteins have been implicated in the control of degradation of cellular regulatory proteins via the ubiquitin-proteasome pathway. Protein degradation by the ubiquitin-proteasome pathway is an essential cellular process of selective removal of proteins from the cell and plays a major role in many cellular processes including signal transduction, transcription and the control of the cell cycle (Hershko et al., Nature Medicine 2000, 6:1073-1081; Joazeiro and Hunter, Science 2000, 289:2061; Pickart, Nature Cell Biology 2000, 2:139-141). A key feature of the ubiquitin-proteasome pathway is the covalent attachment of ubiquitin (ubiquitination) by an enzyme complex to a protein destined for removal. Thereby, the ubiquitin-tagged protein is targeted to the 26S proteasome where it becomes proteolytically degraded. In the central nervous system, proteasome-mediated protein degradation is critical for the breakdown and removal of damaged or misfolded cellular proteins (Alves-Rodrigues et al., Trends Neurosci 1998, 21:516-520). The linkage of ubiquitin to a substrate is carried out by the ubiquitin ligase complex, generally comprising three classes of enzymes in a sequential reaction. Ubiquitin activating enzymes (E1) activate ubiquitin by forming a thioester bond between the E1 enzyme and ubiquitin. Subsequently, a transesterification to a member of the E2 class of enzymes (ubiquitin conjugating enzyme) is carried out. Finally, ubiquitin is transferred from the E2 enzyme to the substrate protein with the assistance of a (E3) ubiquitin ligase. E3 ubiquitin ligases are made up of several components. Specifically, F-box proteins are one of the four components of ubiquitin protein ligases (Deshaies, Annu Rev Cell Dev Biol 1999, 15: 435-467). The function of F-box proteins within the ubiquitin protein ligase complex may be to specifically recruit substrates for ubiquitin conjugation thus conferring substrate specificity to the degradation process. A possible involvement of the human homolog of the *C. elegans* F-box protein Sel-10 in Alzheimer's disease has been disclosed recently (WO 0075328). Sel-10 belongs to a subfamily of F-box proteins which is characterized by multiple WD-40 repeats. This subfamily of F-box proteins is referred to as "Fbw" (for a detailed discussion of the domain structure of F-box proteins, giving rise to altogether three subfamilies of F-box proteins, refer to Winston et al., Current Biology 1999, 9:1180-1182). The subfamily referred to as "Fbx" lacks known protein-interaction domains.

A clearly distinguishable subfamily of F-box proteins contains a C-terminal leucine-rich repeat region in addition to the N-terminally situated F-box motif. Members of this subfamily of the F-box proteins are referred to as F-box and leucine-rich repeat proteins, "Fbl". The gene coding for the human F-box and leucine-repeat protein FBL2 was independently identified by several groups (patent applications by Harper and Elledge, W09918989; Chaiur et al., WO0012679; Zhang et al., W00075184). Exemplary nucleotide sequences of the human FBL2 gene have been deposited in the NCBI Genbank database (accession numbers: NM_012157 (curated version), AF176518 and AF174589 (previous versions)). An examplary amino acid sequence of the corresponding FBL2 protein is referenced in the NCBI Genbank database under the accession number NP_036289 (curated version). Further entries into the database for the FBL2 protein can be found under the NCBI Genbank accession numbers AAF04510 and AAF03128. The rat homolog of the human FBL2 gene exhibits a high degree of similarity to its human counterpart on the amino-acid sequence level (Ilyin et al., FEBS Lett 1999, 459:75-79). The rat FBL2 gene is expressed ubiquitously but with varying amounts among different adult rat tissues. Three different sizes of rat mRNA transcripts were revealed by Northern blot analysis. Whereas the low-sized transcript was predominantly expressed in spleen, thymus and testis, the high molecular weight transcripts were preferably expressed in skeletal muscle, heart and brain. A GFP fusion protein of rat FBL2 expressed in human osteosarcoma U-2OS cells could be localized to punctuate foci in the cytoplasm mainly in the vicinity of the nucleus.

To date, no experiments have been disclosed that demonstrate a relationship between the dysregulation of expression of a gene coding for an F-box and leucine-rich repeat protein, in particular FBL2, and the pathology of neurodegenerative diseases, in particular AD. Such a link, as disclosed in the present invention, offers new ways, inter alia, for the diagnosis and treatment of these diseases.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" as used herein further refer to gene expression levels or gene activity. Gene expression can be defined as the utilization of the information contained in a gene by transcription and translation leading to the production of a gene product. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or lipidation, or by altered signal peptide cleavage or other types of maturation cleavage. These processes may occur post-translationally. Fragments, derivatives, or variants of the F-box and leucine-rich repeat protein FBL2 may include, but are not limited to, a functional F-box domain or other functional modules, such as leucine-repeat modules, contained within the polypeptide sequence of FBL2. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in enzyme activity, a change in binding characteristics, or any other change or alteration in the biological, functional, or immunological properties of said translation product of a gene. The terms "agent", "reagent", or "compound" refer to any substance, chemical, composition, or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix.

The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of an F-box and leucine-rich repeat protein, in particular FBL2. "Variants" include, for example, proteins with conservative amino acid substitutions in highly conservative regions. "Proteins and polypeptides" of the present invention include variants, fragments and chemical derivatives of the protein comprising the amino acid sequences of FBL2. They can include proteins and polypeptides which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. In the present invention, the terms "risk", "susceptibility", and "predisposition" are tantamount and are used with respect to the probability of developing Alzheimer's disease. The term 'AD' shall mean Alzheimer's disease.

In one aspect, the invention features a method of aiding in diagnosing or prognosticating Alzheimer's disease in a subject, or determining whether a subject is at increased risk of developing said disease, comprising:

determining a level of

(i) a transcription product of a gene coding for the F-box protein FBL2, and/or
(ii) a translation product of a gene coding for the F-box protein FBL2, and/or
(iii) a variant of said transcription or translation product, which comprises a sequence that has at least 80% sequence identity with the FBL2 sequence,

in a sample obtained from said subject, wherein said sample is derived from brain tissue,
and comparing said level to a reference value representing a known disease or health status, thereby diagnosing or prognosticating said disease in said subject, or determining whether said subject is at increased risk of developing said Alzheimer's disease.

The disclosure also relates to the construction and the use of primers and probes which are unique to the nucleic acid sequences, or fragments, or derivatives, or variants thereof, as disclosed in the present invention. Oligonucleotide primers and/or probes can be labeled specifically with fluorescent, bioluminescent, magnetic, or radioactive substances. The invention further relates to the detection and the production of said nucleic acid sequences, or fragments, or derivatives, or variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for

Alzheimer's disease. Thus, the disclosure provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with Alzheimers disease. This feature has utility for developing rapid DNA-based diagnostic tests, preferably also in the format of a kit.

In a further aspect, the invention features a method of aiding in monitoring the progression of Alzheimer's disease in a subject, comprising determining levels of a substance as defined in claim 1 (i) to (iii) in a series of samples obtained from said subject over a period of time, wherein said samples are derived from brain tissue.

Said level is compared to a reference value representing a known disease or health status. Thereby, the progression of said disease in said subject is monitored.

In still a further aspect, the invention features a method of aiding in evaluating a treatment for Alzheimer's disease, comprising determining levels of a substance as defined in claim 1 (i) to (iii) in samples obtained from a subject being treated for said disease before and after treatment, wherein said samples are derived from brain tissue.

Said levels are compared to a reference value representing a known disease or health status, thereby evaluating the treatment for said disease.

In the present invention said gene coding for an F-box and leucine-rich repeat protein is the gene coding for FBL2 (comprising nucleotide sequences referenced under NCBI Genbank accession numbers: NM_012157, AF176518, AF174589), and that said F-box and leucine-rich repeat protein is FBL2 (comprising amino acid sequences referenced under NCBI Genbank accession number: NP_036289). It should be understood that, within the scope of the instant invention, the referenced nucleotide and amino acid sequences are of examplary nature, and that a gene coding for FBL2, and the corresponding translation products thereof, also comprise fragments, derivatives, and variants of said nucleotide and amino acid sequences. Further currently known members of the subfamily of human genes coding for F-box and leucine-rich repeat proteins and/or members of the subfamily of human F-box and leucine-rich repeat proteins, and/or fragments thereof, are listed in the following with examplary referencing of their corresponding NCBI Genbank / EMBL accession numbers: FBL1 (Skp2), U33761; FLR1, AF142481; FBL3, AF186273, AK001271; FBL3a, AF129532, FBL3b, AF129533; FBL4, AF174590, AF176699, AF199420; FBL5, AF174591, AF176700, AF199355, BC030656; FBL6, AF174592, AF199356; FBL7, AF174593; FBL9, AF176701; FBL10, AK027692; FBL11, BC001203; human homolog to mouse FBL8, AK002140; human homolog to mouse FBL12, AK000195, BC001586, AK027004; hypothetical 43.3 kDa protein, AL133602; P45SKP2-like protein, AF157323; cDNA FLJ20146, AK000153; cDNA FLJ10576, AK001438; FLJ00115 protein, AK024505; cDNA FLJ22888, AK026541; novel protein BA18114.4, AL121928.

The present invention discloses the detection, differential expression and regulation of a gene coding for the F-box and leucine-rich repeat protein FBL2, in specific brain regions of AD patients. Consequently, the gene coding for an F-box and leucine-rich repeat protein FBL2 and its corresponding gene products may have a causative role in the regional selective neuronal degeneration typically observed in AD. Alternatively, a gene coding for an F-box and leucine-rich repeat protein FBL2 and its corresponding gene products may confer a neuroprotective function to the remaining surviving nerve cells. Based on these disclosures, the present invention has utility for the diagnostic evaluation and prognosis as well as for the identification of a predisposition to AD. Furthermore, the present invention provides in vitro methods for the diagnostic monitoring of patients undergoing treatment for such a disease.

The sample to be analyzed and determined is selected from the group comprising brain tissue. The sample can also comprise cerebrospinal fluid. Preferably, the in vitro methods of diagnosis, prognosis, monitoring the progression or evaluating a treatment for a neurodegenerative disease, according to the instant invention, are pacticed *ex corpore,* and such methods relate to samples, for instance, body fluids or cells, removed, collected, or isolated from a subject or patient.

In further preferred embodiments, said reference value is that of a level, or an activity, or both said level and said activity of (i) a transcription product of a gene coding for an F-box and leucine-rich repeat protein FBL2, and/or of (ii) a translation product of a gene coding for an F-box and leucine-rich repeat protein FBL2, and/or of (iii) variant of said transcription or translation product which comprises a sequence that has at least 80% sequence identity with the FBL2 sequence in a sample from a subject not suffering from said disease.

In preferred embodiments, an alteration in the level and/or activity of a transcription product of a gene coding for an F-box and leucine-rich repeat protein FBL2 and/or a translation product of a gene coding for an F-box and leucine-rich repeat protein FBL2, and or derivative, thereof, in a sample cell, or tissue, or fluid, from said subject relative to a reference value representing a known health status indicates a diagnosis, or prognosis, or increased risk of becoming diseased with Alzheimer's disease.

In preferred embodiments, measurement of a level of transcription products of a gene coding for an F-box and leucine-rich repeat protein FBL2 is performed in a sample from a subject using a quantitative PCR-analysis with primer combinations to amplify said gene-specific sequences from cDNA obtained by reverse transcription of RNA extracted from a sample of a subject. A Northern blot with probes specific for said gene can also be applied. It might further be preferred to measure transcription products by means of chip-based micro-array technologies. These techniques are known to those of ordinary skill in the art (see Sambrook and Russell, Molecular Cloning: A Laboratory Manual, Cold Spring Harbor Laboratory Press, Cold Spring Harbor, New York, 2000).

Furthermore, a level and/or activity of a translation product of a gene coding for an F-box and leucine-rich repeat protein FBL2, and/or fragment, or derivative, or variant of said translation product, can be detected using an immunoassay, an activity assay, and/or binding assay. These assays can measure the amount of binding between said translation product and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, Antibodies: A Laboratory Manual, Cold Spring Harbor Laboratory Press, Cold Spring Harbor, New York, 1999). All these detection techniques may also be employed in the format of microarrays, protein-arrays, antibody microarrays, or protein-chip based technologies.

In one embodiment of the present invention, it might be preferred to determine an enzymatic activity of a translation product of a gene coding for an F-box and leucine-rich repeat protein FBL2, and/or a fragment, or derivative, or variant of said translation product. Specifically, due to an association of F-box and leucine-rich repeat proteins with the ubiquitin-conjugating system, it might be preferred to determine an enzymatic activity of a translation product of a gene coding for an F-box and leucine-rich repeat protein, and/or a fragment, or derivative, or variant of said translation product in an assay for ubiquitin-conjugating enzyme activity, specifically in an assay for E3 ubiquitin-ligase activity. E3 ubiquitin-ligase assays are well known. For instance, such an activity can be determined in a reconstituted system in yeast or in cell-free systems comprising purified or isolated E1 enzyme, E2 enzyme, a translation product of a gene coding for an F-box and leucine-rich repeat protein, and/or a fragment, or derivative, or variant of said translation product, ubiquitin, and an ATP regenerating system (Feldman et al., Cell, 1997; 91:221, Sears et al., J Biol Chem, 1998, 273: 1409; US5976849, W00175145).

In a preferred embodiment, the level, or the activity, or both said level and said activity of (i) a transcription product of a gene coding for an F-box and leucine-rich repeat protein FBL2, and/or of (ii) a translation product of a gene coding for an F-box and leucine-rich repeat protein FBL2, and/or of (iii) a variant of said transcription or translation product which comprises a sequence that has at least 80% sequence identity with the FBL2 sequence in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In another aspect of the disclosure, the method comprises the application of per se known methods of gene therapy and/or antisense nucleic acid technology to administer said agent or agents. In general, gene therapy comprises several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, Acc Chem Res 1993, 26:274-278 and Mulligan, Science, 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3:743-748).

In particular, the disclosure features a method of treating or preventing Alzheimer's disease by means of antisense nucleic acid therapy, i.e. the down-regulation of an inappropriately expressed or defective gene by the introduction of antisense nucleic acids or derivatives thereof into certain critical cells (see e.g. Gillespie, DN&P 1992, 5: 389-395; Agrawal and Akhtar, Trends Biotechnol 1995, 13:197-199; Crooke, Biotechnology 1992, 10:882-6). Apart from hybridization strategies, the application of ribozymes, i.e. RNA molecules that act as enzymes, destroying RNA that carries the message of disease has also been described (see e.g. Barinaga, Science 1993, 262:1512-1514). In preferred embodiments, the subject to be treated is a human, and therapeutic antisense nucleic acids or derivatives thereof are directed against a human F-box and leucine-rich repeat protein gene, particularly the FBL2 gene. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligodeoxynucleotides are known to those of skill in the art (see e.g. Wickstrom, Trends Biotechnol, 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of double-stranded RNA, known variously as RNA interference (RNAi), can be another effective approach for nucleic acid therapy (Hannon, Nature 2002, 418:244-251).

In further aspects of the disclosure, the method of treatment comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection or liposomal mediated transfection.

In the disclosure, said agent for treating and preventing AD is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector. Said agent, particularly a therapeutic protein, can further be administered to said subject by a process comprising the injection or the systemic administration of a fusion protein, said fusion protein being a fusion of a protein transduction domain with said agent.

Methods of treatment, according to the present disclosure, comprise the application of therapeutic cloning, transplantation, and stem cell therapy using embryonic stem cells or embryonic germ cells and neuronal adult stem cells, combined with any of the previously described cell- and gene therapeutic methods. Stem cells may be totipotent or pluripotent. They may also be organ-specific. Strategies for repairing diseased and/or damaged brain cells or tissue comprise (i) taking donor cells from an adult tissue. Nuclei of those cells are transplanted into unfertilized egg cells from which the genetic material has been removed. Embryonic stem cells are isolated from the blastocyst stage of the cells which underwent somatic cell nuclear transfer. Use of differentiation factors then leads to a directed development of the stem cells to specialized cell types, preferably neuronal cells (Lanza et al., Nature Medicine 1999, 9: 975-977), or (ii) purifying adult stem cells, isolated from the central nervous system, or from bone marrow (mesenchymal stem cells), for in vitro expansion and subsequent grafting and transplantation, or (iii) directly inducing endogenous neural stem cells to proliferate, migrate, and differentiate into functional neurons (Peterson DA, Curr. Opin. Pharmacol. 2002, 2: 34-42). Adult neural stem cells are of great potential for repairing damaged or diseased brain tissues, as the germinal centers of the adult brain are free of neuronal damage or dysfunction (Colman A, Drug Discovery World 2001, 7: 66-71).

In preferred aspects, the subject for treatment or prevention, according to the present disclosure, can be a human, an experimental animal, e.g. a mammal, a mouse, a rat, a fish, a fly, or a worm; a domestic animal, or a non-human primate. The experimental animal can be an animal model e.g. a transgenic mouse with an AD-type neuropathology.

Strategies and techniques for the generation and construction of such an animal are known to those of ordinary skill in the art (see e.g. Capecchi, Science, 1989, 244:1288-1292 and Hogan et al., 1994, Manipulating the Mouse Embryo: A Laboratory Manual, Cold Spring Harbor Laboratory Press, Cold Spring Harbor, New York). It is preferred to make use of such a recombinant non-human animal as an animal model for investigating AD.

Said recombinant, non-human animal comprises a non-native gene sequence coding for the F-box and leucine-rich repeat protein FBL2, or a fragment, or derivative, or variant thereof.

In another aspect, the invention features an assay for screening for a modulator of AD said method comprising:

(a) contacting a cell with a test compound;
(b) measuring the level of one or more substances selected from the group consisting of

(i) a gene coding for the F-box protein FBL2, and/or
(ii) a transcription product of a gene coding for the F-box protein FBL2, and/or
(iii) a translation product of a gene coding for the F-box protein FBL2, and /or
(iv) a variant of (i) to (iii), which comprises a sequence that has at least 80% sequence identity with the FBL2 sequence;

(c) measuring the level of one or more substances recited in (i) to (iv) in a control cell not contacted with said test compound; and
(d) comparing the levels of the substance in the cells of step (b) and (c), wherein an alteration in the level of substances in the contacted cells indicates that the test compound is a modulator of said diseases or disorders.

In another aspect, the invention features a method of screening for a modulator of Alzheimer's disease, said method comprising:

(a) taking a non-human test animal which is predisposed to developing or has already developed symptoms of Alzheimer's disease to which a test compound has been administered
(b) measuring the level of one or more substances recited in (i) to (iv) in claim 7 in a sample obtained from said animal, wherein said sample is derived from brain tissue in said non-human test animal;
(c) measuring the level of one or more substances recited in (i) to (iv) in claim 7 in a matched non-human control animal which is predispose to developing or has already developed symptoms of Alzheimer's disease and to which non-human animal no such test compound has been administered in a sample obtained from said animal, wherein said sample is derived from brain tissue; and
(d) comparing the level of the substance in the non-human animals of step (b) and (c), wherein an alteration in the level of substances in the non-human test animal indicates that the test compound is a modulator of said disease.

Said test animal and/or said control animal is a recombinant, non-human animal which expresses an F-box and leucine-rich repeat protein FBL2, or a variant of said protein, which comprises a sequence that has at least 80% sequence identity with the FBL2 sequence under the control of a transcriptional regulatory element which is not the native gene transcriptional control regulatory element.

In another aspect, the present disclosure provides for an assay for testing a compound, preferably for screening a plurality of compounds, for inhibition of binding between a ligand and an F-box and leucine-rich repeat protein FBL2, or a fragment, or derivative, or variant thereof. Said screening assay comprises the steps of (i) adding a liquid suspension of said F-box and leucine-rich repeat protein, or a fragment, or derivative, or variant thereof, to a plurality of containers, and (ii) adding a compound, preferably a plurality of compounds, to be screened for said inhibition to said plurality of containers, and (iii) adding detectable ligand, preferably fluorescently detectable ligand, to said containers, and (iv) incubating the liquid suspension of said F-box and leucine-rich repeat protein, or said fragment, or derivative, or variant thereof, and said compounds, and said detectable ligand, and (v) measuring the amounts of detectable ligand or fluorescence associated with said F-box and leucine-rich repeat protein, or with said fragment, or derivative, or variant thereof, and (vi) determining the degree of inhibition by one or more of said compounds of binding of said ligand to said F-box and leucine-rich repeat protein, or said fragment, or derivative, or variant thereof. Instead of utilizing a fluorescently detectable label, it might in some aspects be preferred to use any other detectable label known to the person skilled in the art, e.g. radioactive label, and detect it accordingly. Said method may be useful for the identification of novel compounds as well as for evaluating compounds which have been improved or otherwise optimized in their ability to inhibit the binding of a ligand to an F-box and leucine-rich repeat protein, or a fragment, or derivative, or variant thereof.

In another aspect, the invention features an assay for testing a compound, preferably for screening a plurality of compounds, to determine the degree of binding of said compound or compounds to an F-box and leucine-rich repeat protein FBL2, or to a fragment, or derivative, or variant thereof. Said screening assay comprises the steps of (i) adding a liquid suspension of said F-box and leucine-rich repeat protein, or a fragment, or derivative, or variant thereof, to a plurality of containers, and (ii) adding a detectable compound, preferably a plurality of detectable compounds, in particular fluorescently detectable compounds, to be screened for said binding to said plurality of containers, and (iii) incubating the liquid suspension of said F-box and leucine-rich repeat protein, or said fragment, or derivative, or variant thereof, and said detectable compound, preferably said plurality of detectable compounds, and (iv) measuring the amounts of detectable compound or fluorescence associated with said F-box and leucine-rich repeat protein, or with said fragment, or derivative, or variant thereof, and (v) determining the degree of binding by one or more of said compounds to said F-box and leucine-rich repeat protein, or said fragment, or derivative, or variant thereof. In this type of assay it might be preferred to use a fluorescent label. However, any other type of detectable label might also be employed. Said method may be useful for the identification of novel compounds as well as for evaluating compounds which have been improved or otherwise optimized in their ability to bind to the F-box and leucine-rich repeat protein FBL2.

In all types of assays disclosed herein, the F-box and leucine-rich repeat protein FBL2 is studied and used.

The present invention features the ex vivo use of a protein molecule, said protein molecule being a translation product of a gene as defined in claim 6, as a diagnostic target for detecting Alzheimer's disease.

The present invention further features the ex vivo use of a protein molecule, said protein molecule being a translation product of a gene as defined in claim 6, as a screening target for reagents or compounds preventing, or treating, or ameliorating Alzheimer's disease.

The present invention features the use of an antibody which is specifically immunoreactive with an immunogen, wherein said immunogen is a translation product of a gene coding for an F-box and leucine-rich repeat protein FBL2 as defined above for detecting the pathological state of a cell in a brain tissue sample obtained from a subject, comprising immunocytochemical staining of said cell with said antibody, wherein an altered degree of staining, or an altered staining pattern in said cell compared to a cell representing a known health status indicates a pathological state of said cell, which pathological state relates to Alzheimer's disease. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof. Antibodies of the present invention are useful, for instance, in a variety of diagnostic and therapeutic methods involving detecting translation products of a gene coding for an F-box and feucine-rich repeat protein.

Said antibodie is used for detecting the pathological state of a cell in a sample from a subject, comprising immunocytochemical staining of said cell with said antibody, wherein an altered degree of staining, or an altered staining pattern in said cell compared to a cell representing a known health status indicates a pathological state of said cell. The pathological state relates to AD. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

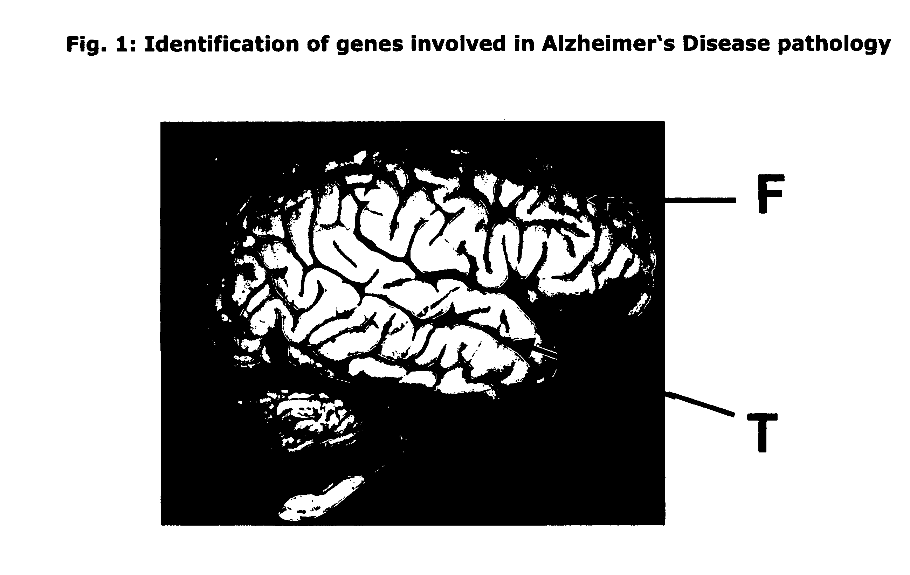

Figure 1 depicts the brain regions with selective vulnerability to neuronal loss and degeneration in AD. Primarily, neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions. In contrast, neurons within the frontal cortex, the occipital cortex, and the cerebellum remain largely intact and preserved from neurodegenerative processes in AD. Brain tissues from the frontal cortex (F) and the temporal cortex (T) of AD patients and healthy, age-matched control individuals were used for the herein disclosed examples. For illustrative purposes, the image of a normal healthy brain was taken from a publication by Strange (Brain Biochemistry and Brain Disorders, Oxford University Press, Oxford, 1992, p.4).

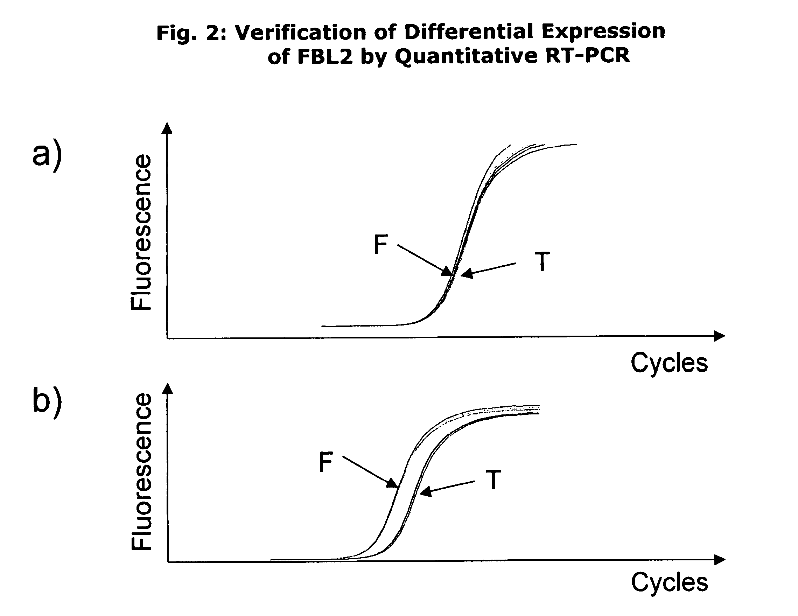

Figure 2 illustrates the verification of the differential expression of the FBL2 gene by quantitative RT-PCR analysis. Quantification of RT-PCR products from RNA samples collected from the frontal cortex (F) and temporal cortex (T) of healthy, age-matched control individuals (Fig 2a) and AD patients (Fig 2b) was performed by the LightCycler rapid thermal cycling technique. The data were normalized to the combined average values of a set of standard genes which showed no significant differences in their gene expression levels. Said set of standard genes consisted of genes for the ribosomal protein S9, the transferrin receptor, GAPDH, and beta-actin. The figure depicts the kinetics of amplification by plotting the cycle number against the amount of amplified material as measured by its fluorescence. The amplification kinetics of FBL2 cDNA from both the frontal and temporal cortices of a normal control individual during the exponential phase of the reaction overlap, whereas in Alzheimer's disease there is a significant separation of the curves for the samples derived from frontal and temporal cortex, which is indicative of an up-regulation of FBL2 gene expression in frontal cortex relative to temporal cortex.

Table 1 lists the gene expression levels in the frontal cortex relative to the temporal cortex for the FBL2 gene in four AD patients (1.84 to 5.44 fold) and four healthy, age-matched control individuals (0.67 to 1.45 fold). The values shown are reciprocal values according to the formula described in present invention.

EXAMPLE I:

Brain tissue dissection from patients with Alzheimer's disease:

Brain tissues from AD patients and age-matched control subjects were collected on average within 5 hours post-mortem and immediately frozen on dry ice. Sample sections from each tissue were fixed in paraformaldehyde for subsequent histopathological confirmation of the diagnosis. Brain areas for differential expression analysis were identified (see Fig. 1) and stored at minus 80°C until RNA extractions were performed.

(i) Isolation of total mRNA:

Total RNA was extracted from post-mortem brain tissue by using the RNeasy kit (Qiagen) according to the manufacturer's protocol. The quality of the prepared RNA was determined by formaldehyde agarose gel electrophoresis and Northern blotting according to standard procedures (Sambrook and Russell, Molecular Cloning: A Laboratory Manual, Cold Spring Harbor Laboratory Press, Cold Spring Harbor, New York, 2000). The accurate RNA concentration and RNA quality were also determined with the DNA LabChip system using the Agilent 2100 Bioanalyzer (Agilent Technologies). For additional quality testing of the prepared RNA, i.e. testing for partial degradation and genomic DNA contamination, specifically designed intronic GAPDH oligonucleotides and genomic DNA as reference control were utilized to generate a melting curve with the LightCycler technology as described in the protocol supplied by the manufacturer (Roche).

(iii) cDNA synthesis and identification of differentially expressed genes by suppressive subtractive hybridization: This technique compares two populations of mRNA and provides clones of genes that are expressed in one population but not in the other. The applied technique was described in detail by Diatchenko et al. (Proc Natl Acad Sci USA 1996, 93:6025-30). In the present invention, mRNA populations derived from different brain regions of AD patients were compared. Specifically, mRNA of the frontal cortex was subtracted from mRNA of the inferior temporal cortex. The necessary reagents were taken from the PCR-Select cDNA subtraction kit (Clontech), and all steps were performed as described in the manufacturer's protocol. Specifically, 2µg mRNA each were used for first-strand and second-strand cDNA synthesis. After Rsal-digestion and adaptor ligation, hybridization of tester and driver was performed for 8 hours (first hybridization) and 15 hours (second hybridization) at 68°C. Two PCR steps were performed to amplify differentially expressed genes (first PCR: 27 cycles of 94°C and 30 sec, 66°C and 30 sec, and 72°C and 1.5 min; nested PCR: 12 cycles of 94°C and 30 sec, 66°C and 30 sec, and 72°C and 1.5 min) using adaptor specific primers (included in the subtraction kit) and 50x Advantage Polymerase Mix (Clontech). Efficiencies of Rsal-digestions, adaptor ligations and subtractive hybridizations were checked as recommended in the kit. Subtracted cDNAs were inserted into the pCR-vector and transformed into E.coli INVαF' cells (Invitrogen). To isolate individual cDNAs of the subtracted library, single bacterial transformants were incubated in 100 µl LB (with 50 µg/ml ampicillin) at 37°C for at least 4 hours. Inserts were PCR amplified (95°C and 30 sec, 68°C and 3 min for 30 cycles) in a volume of 20 $\mu$l containing 10 mM Tris-HCl pH 9.0, 1.5 mM MgCl$_2$, 50 mM KCl, 200 $\mu$M dNTP, 0.5 $\mu$M adaptor specific primers (included in the subtraction kit), 1.5 Units Taq polymerase (Pharmacia Biotech), and 1$\mu$l of bacterial culture. An aliquot of the mixture (1.5 $\mu$l) containing 3 $\mu$l PCR amplified inserts and 2 $\mu$l 0.3 N NaOH / 15% Ficoll were spotted onto a positively charged nylon membrane (Roche). In this way, hundreds of spots were arrayed on duplicate filters for subsequent hybridization. The differential screening step consisted of hybridizations of the subtracted library with itself to minimize background (Wang and Brown, Proc Natl Acad Sci USA 1991, 88:11505-9). The probes were made of the nested PCR product of the subtraction following the instructions of the Clontech subtraction kit. Labeling with digoxigenin was performed with the DIG DNA Labeling Kit (Roche). Hybridizations were carried out overnight in DIG Easy HYB (Roche) at 43°C. The filters were washed twice in 2 x SSC / 0.5 % SDS at 68°C for 15 min and twice in 0.1 x SSC / 0.5 % SDS at 68°C for 15 min, and subjected to detection using anti-DIG-AP conjugates and CDP-Star as chemiluminescent substrate according to the instructions of the DIG DNA Detection Kit (Roche). Blots were exposed to Kodak Biomax MR chemiluminescent film at room temperature for several minutes. The nucleotide sequences of clones of interest were obtained using methods well known to those skilled in the art. For nucleotide sequence analysis, alignments, and homology searches, computer algorithms of the University of Wisconsin Genetics Computer Group (GCG) in conjunction with publicly available nucleotide and protein sequence information (NCBI Genbank and EMBL databases) were employed.

(ii) Confirmation of differential expression by quantitative RT-PCR:

Positive corroboration of differential expression of the FBL2 gene was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic rather than an endpoint approach. The ratio of FBL2 cDNA from the frontal cortex versus temporal cortex was determined (relative quantification). First, a standard curve was generated to determine the efficiency of the PCR with specific primers for FBL2 (5'-TACTGGGTGGAGCAGGGTCTT-3' and 5'-GGTCCCTGGAGGTGTATATGACA-3'). PCR amplification (95°C and 1 sec, 56°C and 5 sec, and 72°C and 5 sec) was performed in a volume of 20 $\mu$l containing Lightcycler-DNA-Master-SYBR-Green mix (containing Taq DNA polymerase, reaction buffer, dNTP mix with dUTP instead of dTTP, SYBR Green I dye, and 1 mM MgCl$_2$, Roche), additionally containing 3 mM MgCl$_2$, 0,5 $\mu$M primers, 0,16 $\mu$l TaqStart antibody (Clontech), and 1 $\mu$l of a cDNA dilution series (40, 20, 10, 5, and 1 ng human total brain cDNA, Clontech). Melting curve analysis revealed a single peak at approximately 83°C with no visible primer dimers. Quality and size of the PCR product were determined by agarose gel electrophoresis and DNA LabChip analysis (Agilent 2100 Bioanalyzer, Agilent Technologies). A single band of the expected size 66 bp was observed.

In an analogous manner, the above described PCR protocol (with indicated exceptions) was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTTTGCC-3', (exception: an additional 1 mM MgCl$_2$ was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'-TCTCATCAAGCGTCAGCAGTTC-3' (exception: an additional 1 mM MgCl$_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'- TGGAACGGTGAAGGTGACA-3' and 5'- GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers 5'-CGTCATGGGTGTGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'- GTCGCTGGTCAGTTCGTGATT-3' and 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for FBL2 and the five reference standard genes. The slopes and the intercepts of the standard curves (i.e. linear regressions) were calculated for all genes. In a second step, cDNAs from temporal cortex and frontal cortex were analyzed in parallel and normalized to cyclophilin B. The $C_t$ values were measured and converted to ng total brain cDNA using the corresponding standard curves:

$$10 \wedge ( (C_t \text{ value - intercept}) / \text{slope} ) \quad [\text{ng total brain cDNA}]$$

The values of temporal and frontal cortex FBL2 cDNAs were normalized to cyclophilin B, and the ratio was calculated using the following formula:

$$\text{Ratio} = \frac{\text{FBL2 temporal [ng] / cyclophilin B temporal [ng]}}{\text{FBL2 frontal [ng] / cyclophilin B frontal [ng]}}$$

In a third step, the set of reference standard genes was analyzed in parallel to determine the mean average value of the temporal to frontal ratios of expression levels of the reference standard genes for each individual brain sample. As cyclophilin B was analyzed in step 2 and step 3, and the ratio from one gene to another gene remained constant in different runs, it was possible to normalize FBL2 to the mean average value of the set of reference standard genes instead of normalizing to one single gene alone. The calculation was performed by dividing the ratio shown above by the deviation of cyclophilin B from the mean value of all housekeeping genes. The results of one such quantitative RT-PCR analysis for the FBL2 gene are shown in Fig. 2.

Table 1:

| SAMPLE | $\Delta$ (fold) |
|---|---|
| patient 1 (#16) | 1.98 |
| patient 2 (#10) | 5.44 |
| patient 3 (#11) | 2.23 |
| patient 4 (#14) | 1.84 |
| | |
| control 1 (#10) | 0.97 |
| control 2 (#11) | 0.94 |
| control 3 (# 5) | 1.45 |
| control 4 (# 4) | 0.67 |

**Claims**

1.  A method of aiding in diagnosing or prognosticating Alzheimer's disease in a subject, or determining whether a subject is at increased risk of developing said disease, comprising:

    determining a level of

    (i) a transcription product of a gene coding for the F-box protein FBL2, and/or
    (ii) a translation product of a gene coding for the F-box protein FBL2, and/or
    (iii) a variant of said transcription or translation product, which comprises a sequence that has at least 80% sequence identity with the FBL2 sequence,

    in a sample obtained from said subject, wherein said sample is derived from brain tissue,
    comparing said level to a reference value representing a known disease or health status, and thereby diagnosing or prognosticating said disease,
    whether or determining said subject is at increased risk of developing said disease,

2.  A method of aiding in monitoring the progression of Alzheimer's disease in a subject, comprising determining levels of a substance as defined in claim 1 (i) to (iii)

in a series of samples obtained from said subject over a period of time, wherein said samples are derived from brain tissue,

comparing said levels, and thereby

monitoring the progression of said disease in said subject.

3.  A method of aiding in evaluating a treatment for Alzheimer's disease, comprising determining levels of a substance as defined in claim 1(i) to (iii)

in samples obtained from a subject being treated for said disease before and after treatment, wherein said samples are derived from brain tissue,

comparing said levels, and thereby

evaluating said treatment for said disease.

4.  The method according to any of claims 1 to 3 wherein said reference value is that of a level of a substance as defined in claim 1 (i) to (iii) in a sample obtained from a subject not suffering from Alzheimer's disease, wherein said sample is derived from brain tissue.

5.  Ex vivo Use of at least one reagent which is selected from the group consisting of (i) reagents that selectively detect a transcription product of a gene coding for the F-box protein FBL2 and (ii)

reagents that selectively detect a translation product of a gene coding for the F-box protein FBL2, for diagnosing or prognosticating Alzheimer's disease, in a subject, or determining the propensity or predisposition of a subject to develop such disease.

6.  Use of a recombinant, non-human animal comprising a non-native gene sequence coding for the F-box protein FBL2, or a variant of said protein, which comprises a sequence that has at least 80% sequence identity with the FBL2 sequence, as an animal model for investigating Alzheimer's disease.

7.  An assay for screening for a modulator of Alzheimer's disease, said method comprising:

    (a) contacting a cell with a test compound;
    (b) measuring the level of one or more substances selected from the group consisting of

    (i) a gene coding for the F-box protein FBL2, and/or
    (ii) a transcription product of a gene coding for the F-box protein FBL2, and/or
    (iii) a translation product of a gene coding for the F-box protein FBL2, and /or
    (iv) a variant of (i) to (iii), which comprises a sequence that has at least 80% sequence identity with the FBL2 sequence;

    (c) measuring the level of one or more substances recited in (i) to (iv) in a control cell not contacted with said test compound; and
    (d) comparing the levels of the substance in the cells of step (b) and (c), wherein an alteration in the level of substances in the contacted cells indicates that the test compound is a modulator of said diseases or disorders.

8.  A method of screening for a modulator of Alzheimer's disease, said method comprising

    (a) taking a non-human test animal which is predisposed to developing or has already developed symptoms of Alzheimer's disease to which a test compound has been administered;
    (b) measuring the level of one or more substances recited in (i) to (iv) in claim 7 in a sample obtained from said animal, wherein said sample is derived from brain tissue in said non-human test animal;
    (c) measuring the level of one or more substances recited in (i) to (iv) in claim 7 in a matched non-human control animal which is predisposed to developing or has already developed symptoms of Alzheimer's disease and to which non-human animal no such test compound has been administered in a sample obtained from said animal, wherein said sample is derived from brain tissue; and
    (d) comparing the level of the substance in the non-human animals of step (b) and (c), wherein an alteration in the level of substances in the non-human test animal indicates that the test compound is a modulator of said disease.

9.  The method according to claim 8 wherein said test animal and/or said control animal is a recombinant non-human animal which expresses the F-box protein FBL2, or a variant of said protein, which comprises a sequence that has at least 80% sequence identity with the FBL2 sequence, under the control of a transcriptional control element which is not the native gene transcriptional control element.

10. Ex vivo Use of a protein molecule, said protein molecule being a translation product of a gene as defined in claim 6, as a diagnostic target for detecting Alzheimer's disease.

11. Ex vivo Use of a protein molecule, said protein molecule being a translation product of a gene as defined in claim 6, as a screening target for reagents or compounds preventing, or treating, or ameliorating Alzheimer's disease.

12. Use of an antibody immunoreactive with an immunogen, wherein said immunogen is a translation product of a gene as defined in claim 6, for detecting the pathological state of a cell in a brain tissue sample obtained from a subject, comprising immunocytochemical staining of said cell with said antibody, wherein an altered degree of staining, or an altered staining pattern in said cell compared to a cell representing a known health status indicates a pathological state of said cell, which pathological state relates to Alzheimer's disease.

**Patentansprüche**

1. Verfahren zur Unterstützung der Diagnose oder Prognose der Alzheimer-Krankheit bei einem Patienten oder zum Bestimmen, ob ein Patient ein erhöhtes Risiko hat, diese Krankheit zu entwickeln, umfassend:

   Bestimmen einer Konzentration von:

   (i) einem Transcriptionsprodukt eines Gens, das für das F-Box-Protein FBL2 codiert; und/oder
   (ii) einem Translationsprodukt eines Gens, das für das F-Box-Protein FBL2 codiert; und/oder
   (iii) einer Variante des Transcriptions- oder Translationsprodukts, die eine Sequenz umfasst, die wenigstens 80% Sequenzidentität mit der FBL2-Sequenz aufweist;

   in einer von dem Patienten erhaltenen Probe, wobei die Probe aus Gehirngewebe stammt;
   Vergleichen der Konzentration mit einem Referenzwert, der einen bekannten Krankheits- oder Gesundheitszustand darstellt, und **dadurch**
   Diagnostizieren oder Prognostizieren der Krankheit oder Bestimmen, ob der Patient ein erhöhtes Risiko hat, die Krankheit zu entwickeln.

2. Verfahren zur Unterstützung der Überwachung des Fortschritts von Alzheimer-Krankheit bei einem Patienten, umfassend das Bestimmen von Konzentrationen einer Substanz, wie sie in Anspruch 1 (i) bis (iii) definiert ist;
   in einer Reihe von Proben, die über einen Zeitraum hinweg von dem Patienten erhalten wurden, wobei die Proben aus Gehirngewebe stammen;
   Vergleichen der Konzentrationen und **dadurch** Überwachen des Fortschritts der Krankheit bei dem Patienten.

3. Verfahren zur Unterstützung der Bewertung einer Behandlung auf Alzheimer-Krankheit, umfassend das Bestimmen von Konzentrationen einer Substanz, wie sie in Anspruch 1 (i) bis (iii) definiert ist;
   in Proben, die von einem Patienten, der auf die Krankheit behandelt wird, vor und nach der Behandlung erhalten wurden, wobei die Proben aus Gehirngewebe stammen;
   Vergleichen der Konzentrationen und **dadurch**
   Bewerten der Behandlung auf die Krankheit.

4. Verfahren gemäß einem der Ansprüche 1 bis 3, wobei der Referenzwert der Referenzwert einer Konzentration einer Substanz, wie sie in Anspruch 1 (i) bis (iii) definiert ist, in einer Probe von einem Patienten, der nicht an Alzheimer-Krankheit leidet, ist, wobei die Probe aus Gehirngewebe stammt.

5. Ex-vivo-Verwendung wenigstens eines Reagens, das aus der Gruppe ausgewählt ist, die aus Folgenden besteht: (i) Reagentien, die selektiv ein Transcriptionsprodukt eines Gens, das für das F-Box-Protein FBL2 codiert, nachweisen, und (ii) Reagentien, die selektiv ein Translationsprodukt eines Gens, das für das F-Box-Protein FBL2 codiert, nachweisen, zum Diagnostizieren oder Prognostizieren von Alzheimer-Krankheit bei einem Patienten oder zum Bestimmen der Neigung oder Prädisposition eines Patienten, eine solche Krankheit zu entwickeln.

6. Verwendung eines rekombinanten nichthumanen Tiers, das eine nichtnative Gensequenz umfasst, die für das F-Box-Protein FBL2 oder eine Variante dieses Proteins, die eine Sequenz umfasst, die wenigstens 80% Sequenzidentität mit der FBL2-Sequenz aufweist, codiert, als Tiermodell zur Untersuchung von Alzheimer-Krankheit.

7. Assay zum Suchen nach einem Modulator der Alzheimer-Krankheit, wobei das Verfahren Folgendes umfasst:

(a) In-Kontakt-Bringen einer Zelle mit einer Testverbindung;
(b) Messen der Konzentration von einer oder mehreren Substanzen, die aus der Gruppe ausgewählt sind, die aus Folgenden besteht:

(i) einem Gen, das für das F-Box-Protein FBL2 codiert; und/oder
(ii) einem Transcriptionsprodukt eines Gens, das für das F-Box-Protein FBL2 codiert; und/oder
(iii) einem Translationsprodukt eines Gens, das für das F-Box-Protein FBL2 codiert; und/oder
(iii) einer Variante von (i) bis (iii), die eine Sequenz umfasst, die wenigstens 80% Sequenzidentität mit der FBL2-Sequenz aufweist;

(c) Messen der Konzentration von einer oder mehreren Substanzen, die in (i) bis (iv) genannt sind, in einer Kontrollzelle, die nicht mit der Testverbindung in Kontakt gebracht wurde; und
(d) Vergleichen der Konzentrationen der Substanz in den Zellen der Schritte (b) und (c), wobei eine Änderung der Konzentration der Substanzen in den kontaktierten Zellen anzeigt, dass die Testverbindung ein Modulator der Krankheiten oder Störungen ist.

8. Verfahren zum Suchen nach einem Modulator der Alzheimer-Krankheit, wobei das Verfahren Folgendes umfasst:

(a) Bereitstellen eines nichthumanen Versuchstiers, das prädisponiert ist, Symptome der Alzheimer-Krankheit zu entwickeln, oder diese bereits entwickelt hat und dem eine Testverbindung verabreicht wurde;
(b) Messen der Konzentration von einer oder mehreren Substanzen, die in (i) bis (iv) in Anspruch 7 genannt sind, in einer von dem Tier erhaltenen Probe, wobei die Probe aus Gehirngewebe in dem nichthumanen Versuchstier stammt;
(c) Messen der Konzentration von einer oder mehreren Substanzen, die in (i) bis (iv) in Anspruch 7 genannt sind, in einem passenden nichthumanen Kontrolltier, das prädisponiert ist, Symptome der Alzheimer-Krankheit zu entwickeln, oder diese bereits entwickelt hat, wobei diesem nichthumanen Tier keine solche Testverbindung verabreicht wurde, in einer von dem Tier erhaltenen Probe, wobei die Probe aus Gehirngewebe stammt; und
(d) Vergleichen der Konzentration der Substanz in den nichthumanen Tieren der Schritte (b) und (c), wobei eine Änderung der Konzentration der Substanzen in dem nichthumanen Versuchstier anzeigt, dass die Testverbindung ein Modulator der Krankheit ist.

9. Verfahren gemäß Anspruch 8, wobei das Versuchstier und/oder das Kontrolltier ein rekombinantes nichthumanes Tier ist, das das F-Box-Protein FBL2 oder eine Variante des Proteins, die eine Sequenz umfasst, die wenigstens 80% Sequenzidentität mit der FBL2-Sequenz aufweist, unter der Kontrolle eines Transcriptionskontrollelements, bei dem es sich nicht um das native Transcriptionskontrollelement des Gens handelt, exprimiert.

10. Ex-vivo-Verwendung eines Proteinmoleküls, wobei das Proteinmolekül ein Translationsprodukt eines Gens ist, wie es in Anspruch 6 definiert ist, als diagnostisches Target zum Nachweisen der Alzheimer-Krankheit.

11. Ex-vivo-Verwendung eines Proteinmoleküls, wobei das Proteinmolekül ein Translationsprodukt eines Gens ist, wie es in Anspruch 6 definiert ist, als Screening-Target für Reagentien oder Verbindungen, die die Alzheimer-Krankheit verhindern oder behandeln oder lindern.

12. Verwendung eines Antikörpers, der gegenüber einem Immunogen immunreaktiv ist, wobei das Immunogen ein Translationsprodukt eines Gens ist, wie es in Anspruch 6 definiert ist, zum Nachweis des pathologischen Zustands einer Zelle in einer Gehirngewebeprobe, die von einem Patienten erhalten wurde, umfassend das immunocytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle anzeigt, wobei der pathologische Zustand mit der Alzheimer-Krankheit in Zusammenhang steht.

**Revendications**

1. Procédé pour l'aide au diagnostic ou au pronostic de la maladie d'Alzheimer chez un sujet, ou le fait de déterminer si un sujet présente un risque accru de développement de ladite maladie, le procédé comprenant :

   la détermination du niveau de

   (i) un produit de transcription d'un gène codant la protéine F-box FBL2, et/ou
   (ii) un produit de traduction d'un gène codant la protéine F-box FBL2, et/ou
   (iii) un variant dudit produit de transcription ou de traduction, qui comprend une séquence qui a au moins 80% d'identité avec la séquence de FBL2,

   dans un échantillon obtenu à partir dudit sujet, ledit échantillon étant dérivé de tissu de cerveau,
   la comparaison dudit niveau à une valeur de référence représentant un statut connu d'une maladie ou d'un état de santé, et de ce fait
   l'établissement d'un diagnostic ou un pronostic de ladite maladie,
   ou la détermination du fait de savoir si ledit sujet a un risque accru de développer ladite maladie.

2. Procédé pour l'aide à la surveillance de la progression de la maladie d'Alzheimer chez un sujet, procédé qui comprend la détermination des niveaux d'une substance telle que définie dans la revendication 1 (i) à (iii),
   dans une série d'échantillons obtenus à partir dudit sujet sur une période de temps, dans lequel lesdits échantillons sont dérivés de tissu de cerveau,
   la comparaison desdits niveaux, et de ce fait
   la surveillance de la progression de ladite maladie chez ledit suj et.

3. Procédé pour l'aide à l'évaluation d'un traitement pour la maladie d'Alzheimer, comprenant la détermination des niveaux d'une substance telle que définie dans la revendication 1 (i) à (iii)
   dans des échantillons obtenus à partir d'un sujet qui est traité pour ladite maladie, avant et après le traitement, dans lequel lesdits échantillons sont dérivés de tissu de cerveau,
   la comparaison desdits niveaux, et de ce fait
   l'évaluation dudit traitement pour ladite maladie.

4. Procédé selon l'une quelconque des revendications 1 à 3, dans lequel ladite valeur de référence est celle du niveau d'une substance telle que définie dans la revendication 1 (i) à (iii) dans un échantillon obtenu à partir d'un sujet qui n'est pas atteint de la maladie d'Alzheimer, dans lequel ledit échantillon est dérivé de tissu de cerveau.

5. Utilisation *ex vivo* d'au moins un réactif choisi dans le groupe constitué de (i) des réactifs qui détectent de façon sélective un produit de transcription d'un gène codant la protéine F-box FBL2, et
   (ii) des réactifs qui détectent de façon sélective un produit de traduction d'un gène codant la protéine F-box FBL2, pour le diagnostic ou le pronostic de la maladie d'Alzheimer, chez un sujet, ou la détermination de la propension ou de la prédisposition d'un sujet à développer ladite maladie.

6. Utilisation d'un animal recombinant non-humain comprenant une séquence génique non-native codant la protéine F-box FBL2, ou un variant de ladite protéine, qui comprend une séquence qui a au moins 80% d'identité avec la séquence de FBL2, comme modèle animal pour étudier la maladie d'Alzheimer.

7. Test pour le criblage d'un modulateur de la maladie d'Alzheimer, ledit procédé comprenant :

   (a) le fait de mettre en contact une cellule avec un composé test,
   (b) le fait de mesurer le niveau d'une ou plusieurs substances choisies dans le groupe constitué de

   (i) un gène codant la protéine F-box FBL2, et/ou
   (ii) un produit de transcription d'un gène codant la protéine F-box FBL2, et/ou
   (iii) un produit de traduction d'un gène codant la protéine F-box FBL2, et/ou
   (iv) un variant de (i) à (iii), comprenant une séquence qui a au moins 80% d'identité avec la séquence FBL2 ;

   (c) le fait de mesurer le niveau d'une ou plusieurs substances citées dans (i) à (iv) dans une cellule contrôle qui n'a pas été mise en contact avec ledit composé test ; et

(d) le fait de comparer les niveaux de la substance dans les cellules des étapes (b) et (c), dans lesquelles une altération du niveau des substances dans les cellules en contact indique que le composé test est un modulateur desdites maladies ou troubles.

8. Procédé de criblage pour un modulateur de la maladie d'Alzheimer, ledit procédé comprenant

(a) le fait de prendre un animal-test non-humain qui est prédisposé à développer ou qui a déjà développé des symptômes de la maladie d'Alzheimer, auquel un composé test a été administré,
(b) le fait de mesurer le niveau d'une ou plusieurs substances citées dans (i) à (iv) de la revendication 7, dans un échantillon obtenu à partir dudit animal, ledit échantillon étant dérivé de tissu de cerveau dudit animal-test non-humain,
(c) le fait de mesurer le niveau d'une ou plusieurs substances citées dans (i) à (iv) de la revendication 7, chez un animal-contrôle non-humain apparié qui est prédisposé à développer ou qui a déjà développé des symptômes de la maladie d'Alzheimer, et pour lequel animal, aucun composé-test semblable n'a été administré dans un échantillon obtenu à partir dudit animal, ledit échantillon étant dérivé de tissu de cerveau, et
(d) le fait de comparer le niveau de la substance chez des animaux non-humains dans les étape (b) et (c), dans lesquelles une altération au niveau des substances chez l'animal-test non-humain indique que le composé test est un modulateur de ladite maladie.

9. Procédé selon la revendication 8, dans lequel ledit animal-test et/ou ledit animal-contrôle est un animal non-humain recombinant qui exprime la protéine F-box FBL2, ou un variant de ladite protéine, qui comprend une séquence qui a au moins 80% d'identité avec la séquence FBL2, sous le contrôle d'un élément de contrôle transcriptionnel qui n'est pas l'élément de contrôle transcriptionnel du gène natif.

10. Utilisation *ex vivo* d'une molécule protéique, ladite molécule protéique étant un produit de traduction d'un gène tel que défini dans la revendication 6, comme cible diagnostic pour détecter la maladie d'Alzheimer.

11. Utilisation *ex vivo* d'une molécule protéique, ladite molécule protéique étant un produit de traduction d'un gène tel que défini dans la revendication 6, comme cible de criblage pour des réactifs ou des composés qui préviennent, traitent, ou améliorent la maladie d'Alzheimer.

12. Utilisation d'un anticorps immunoréactif avec un immunogène, ledit immunogène étant un produit de traduction d'un gène tel que défini dans la revendication 6, pour détecter l'état pathologique, dans une cellule d'un échantillon de tissu de cerveau obtenu à partir d'un sujet, comprenant la coloration immunocytochimique de ladite cellule avec ledit anticorps, dans laquelle un degré altéré de coloration, ou un profil altéré de coloration dans ladite cellule, comparé à une cellule représentant un état de santé connu, indique un état pathologique de ladite cellule, ledit état pathologique étant associé à la maladie d'Alzheimer.

Fig. 1: Identification of genes involved in Alzheimer's Disease pathology

Fig. 2: Verification of Differential Expression of FBL2 by Quantitative RT-PCR

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0075328 A
- WO 9918989 A, Harper and Elledge
- WO 0012679 A, Chaiur
- WO 0075184 A, Zhang
- US 5976849 A
- WO 0175145 A
- US 6150173 A

### Non-patent literature cited in the description

- **Vickers et al.** *Progress in Neurobiology,* 2000, vol. 60, 139-165
- **Terry et al.** Annals of Neurology. 1981, vol. 10, 184-192
- **Bai et al.** *Cell,* 1996, vol. 86, 263-274
- **Kipreos ; Pagano.** *Genome Biology,* 2000, vol. 1, 3002.1-3002.7
- **Cenciarelli et al.** *Current Biology,* 1999, vol. 9, 1177-1179
- **Winston et al.** *Current Biology,* 1999, vol. 9, 1180-1182
- **Hershko et al.** *Nature Medicine,* 2000, vol. 6, 1073-1081
- **Joazeiro ; Hunter.** *Science,* 2000, vol. 289, 2061
- **Pickart.** *Nature Cell Biology,* 2000, vol. 2, 139-141
- **Alves-Rodrigues et al.** *Trends Neurosci,* 1998, vol. 21, 516-520
- **Deshaies.** *Annu Rev Cell Dev Biol,* 1999, vol. 15, 435-467
- **Ilyin et al.** *FEBS Lett,* 1999, vol. 459, 75-79
- **Sambrook ; Russell.** Molecular Cloning: A Laboratory Manual. Cold Spring Harbor Laboratory Press, 2000
- **Harlow ; Lane.** Antibodies: A Laboratory Manual. Cold Spring Harbor Laboratory Press, 1999
- **Feldman et al.** *Cell,* 1997, vol. 91, 221
- **Sears et al.** *J Biol Chem,* 1998, vol. 273, 1409
- **Behr.** *Acc Chem Res,* 1993, vol. 26, 274-278
- **Mulligan.** *Science,* 1993, vol. 260, 926-931
- **Wolff.** *Curr Opin Neurobiol,* 1993, vol. 3, 743-748
- **Gillespie.** *DN&P,* 1992, vol. 5, 389-395
- **Agrawal ; Akhtar.** *Trends Biotechnol,* 1995, vol. 13, 197-199
- **Crooke.** *Biotechnology,* 1992, vol. 10, 882-6
- **Barinaga.** *Science,* 1993, vol. 262, 1512-1514
- **Wickstrom.** *Trends Biotechnol,* 1992, vol. 10, 281-287
- **Hannon.** *Nature,* 2002, vol. 418, 244-251
- **Lanza et al.** *Nature Medicine,* 1999, vol. 9, 975-977
- **Peterson DA.** *Curr. Opin. Pharmacol.,* 2002, vol. 2, 34-42
- **Colman A.** *Drug Discovery World,* 2001, vol. 7, 66-71
- **Capecchi.** *Science,* 1989, vol. 244, 1288-1292
- **Hogan et al.** Manipulating the Mouse Embryo: A Laboratory Manual. Cold Spring Harbor Laboratory Press, 1994
- **Harlow et al.** Antibodies, A Laboratory Manual. Cold Spring Harbor Laboratory Press
- **Strange.** Brain Biochemistry and Brain Disorders. Oxford University Press, 1992, 4
- **Diatchenko et al.** *Proc Natl Acad Sci USA,* 1996, vol. 93, 6025-30
- **Wang ; Brown.** *Proc Natl Acad Sci USA,* 1991, vol. 88, 11505-9